**Europäisches Patentamt**

**European Patent Office**

**Office européen des brevets**

(19)

(11) Veröffentlichungsnummer : **0 304 757 B1**

(12) # EUROPÄISCHE PATENTSCHRIFT

(45) Veröffentlichungstag der Patentschrift :
16.10.91 Patentblatt 91/42

(51) Int. Cl.⁵ : **C07C 69/54, C07C 67/08**

(21) Anmeldenummer : **88113227.8**

(22) Anmeldetag : **16.08.88**

(54) Verfahren zur Herstellung von Carbonsäureestern.

(30) Priorität : **25.08.87 DE 3728242**

(43) Veröffentlichungstag der Anmeldung :
**01.03.89 Patentblatt 89/09**

(45) Bekanntmachung des Hinweises auf die
Patenterteilung :
**16.10.91 Patentblatt 91/42**

(84) Benannte Vertragsstaaten :
**CH DE ES FR GB IT LI NL**

(56) Entgegenhaltungen :
**DE-A- 2 449 811
DE-A- 2 552 987
DE-A- 2 904 822**

(73) Patentinhaber : **BASF Aktiengesellschaft
Carl-Bosch-Strasse 38
W-6700 Ludwigshafen (DE)**

(72) Erfinder : **Bott, Kaspar, Dr.
Werderstrasse 57
W-6800 Mannheim 1 (DE)**
Erfinder : **Hartmann, Horst
Lindenstrasse 45
W-6737 Boehl-Iggelheim (DE)**
Erfinder : **Guth, Josef
Pfarrer-Friedrich-Strasse 41
W-6700 Ludwigshafen (DE)**

**Beschreibung**

Die vorliegende Erfindung betrifft ein Verfahren zur Herstellung von Carbonsäureestern durch Umsetzung von Carbonsäuren mit niedersiedenden Alkoholen.

Für die Wirtschaftlichkeit einer technischen Produktion von Carbonsäureestern — im folgenden Ester genannt — aus Carbonsäuren und Alkoholen ist es entscheidend, daß man eines der Reaktionsprodukte Ester bzw. Wasser oder beide durch Destillation aus der Reaktionsmischung abtrennen kann, um auf diese Weise das Estergleichgewicht möglichst vollständig in die gewünschte Richtung zu verschieben. Ein solches Verfahren stößt jedoch auf Schwierigkeiten, wenn man niedersiedende Alkohole wie Methanol oder Ethanol als Reaktionspartner einsetzt, weil in diesem Fall der betreffende Alkohol oder eine Mischung desselben mit dem gebildeten Ester vielfach die am niedrigsten siedende Komponente der Reaktionsmischung darstellt. Außerdem sind die niedersiedenden Alkohole zum Auskreisen des Wassers durch Schleppdestillation wenig geeignet, weil sie in Gegenwart des letzteren keine zwei Flüssigphasen auszubilden vermögen, aus denen man das Wasser leicht abtrennen kann. Die hiermit verbundenen Probleme lassen sich dadurch umgehen, daß man für das Ausschleusen des Wassers ein Umwälzungsmittel in der Destillation verwendet. Die Verwendung derartiger Umwälzungsmittel ist in der Fachliteratur ausführlich beschrieben, beispielsweise in Houben-Weyl, Methoden der Organischen Chemie, 4. Auflage, Band VIII, Seite 522 und folgende.

Die Verwendung eines Umwälzungsmittels bei der Veresterung von Carbonsäuren mit niedersiedenden Alkoholen ist im allgemeinen mit zusätzlichen Trennoperationen verbunden, die sich auf die Wirtschaftlichkeit des Verfahrens nachteilig auswirken können.

Es stellte sich daher die Aufgabe, Carbonsäuren mit niedersiedenden Alkoholen wie Methanol oder Ethanol möglichst vollständig zu verestern, ohne daß ein nicht an der Reaktion beteiligter Stoff für die Abtrennung des Wassers eingesetzt werden muß.

Diese Aufgabe wird erfindungsgemäß dadurch gelöst, daß das Reaktionsgemisch einer 1. Destillationskolonne zugeführt wird, die Reaktionskomponenten Carbonsäureester und Wasser und der nicht umgesetzte Alkohol als Kopfprodukt abgezogen und einer 2. Destillationskolonne zugeführt werden, wobei aus dieser

a)  als Kopfprodukt der Alkohol oder ein azeotrop siedendes Gemisch aus Alkohol und Wasser bzw. aus Alkohol und Ester entnommen und in die Veresterungsreaktion zurückgeführt wird,

b)  aus dem Abtriebsteil ein flüssiger Seitenabzug, bestehend aus Carbonsäureester und Wasser, entnommen und in einem Phasentrenngefäß in Wasser und Carbonsäureester getrennt wird und der Carbonsäureester unterhalb der Seitenentnahme der Destillationskolonne wieder zugeführt wird, und

c)  als Sumpfprodukt der Carbonsäureester entnommen wird.

Weitere erfindungsgemäße Merkmale der Erfindung sind Gegenstand der Unteransprüche.

Das vorliegende Verfahren läßt sich besonders vorteilhaft in kontinuierlicher Fahrweise betreiben und kann darüber hinaus auch auf die Veresterung solcher Carbonsäuren ausgedehnt werden, die als Verunreinigung noch kleine Mengen einer niedriger siedenden Komponente enthalten. Ein Beispiel hierfür ist die Herstellung von Halogencarbonsäuremethylestern aus Methanol und einer technischen Halogencarbonsäure, die mit der halogenfreien Carbonsäure verunreinigt ist. In diesem Fall reichert sich die verunreinigende Komponente als Carbonsäuremethylester im Kopfprodukt der 2. Destillationskolonne an und kann dort zusammen mit dem rückgeführten Methanol abgezogen werden.

Geeignete Alkohol/Carbonsäure-Paare, auf die das erfindungsgemäße Verfahren anwendbar ist, sind beispielsweise

Methanol/Methacrylsäure
Methanol/Bromessigsäure
Methanol/Brompropionsäure
Methanol/Buttersäure
Methanol/2-Chlorbuttersäure
Methanol/4-Chlorbuttersäure
Methanol/2-Bromisobuttersäure
Methanol/Thioglykolsäure
Methanol/Crotonsäure
Methanol/Sorbinsäure
Methanol/2-Mercaptopropionsäure
Methanol/Mercaptobuttersäure
Ethanol/2-Chlorpropionsäure
Ethanol/2-Brompropionsäure
Ethanol/2-Chlorbuttersäure
Ethanol/2-Chlorisobuttersäure
Ethanol/4-Chlorbuttersäure
Ethanol/2-Bromisobuttersäure

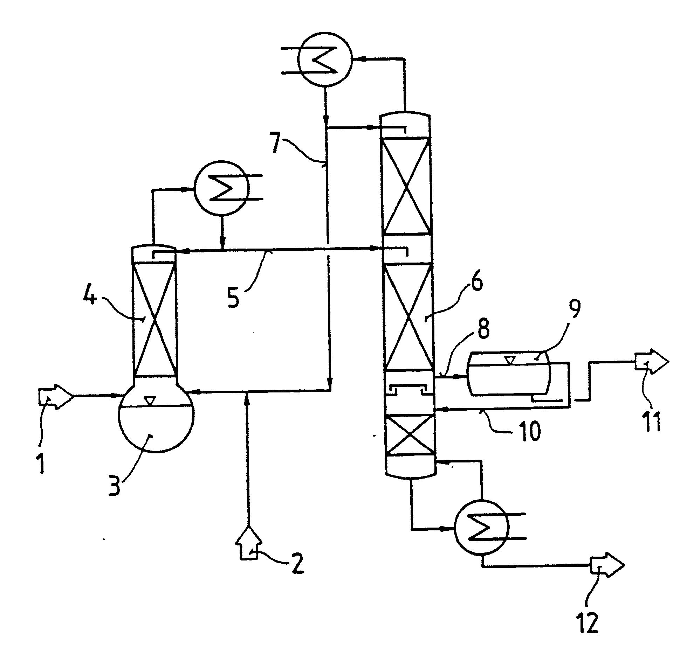

Das erfindungsgemäße Verfahren wird im folgenden anhand der Abbildung näher beschrieben.

Die mit 1 bezeichnete Carbonsäure $R^1\text{-}CO_2H$ und der mit 2 bezeichnete niedersiedende Alkohol $R^2\text{-}OH$ werden in dem Reaktionsgefäß 3 zur Umsetzung gebracht. Aus der dabei resultierenden Reaktionsmischung wird in einer Verstärkungskolonne 4 das mit 5 bezeichnete Gemisch aus Ester $R^1\text{-}CO_2R^2$, Wasser und nicht umgesetztem Alkohol $R^2OH$ über Kopf abgetrennt und der Destillationskolonne 6 zugeführt, in der die weitere Trennung wie folgt abläuft :

a)  Als Kopfprodukt 7 wird Alkohol oder ein azeotrop siedendes Gemisch aus Alkohol und Wasser bzw. aus Alkohol und Ester abgetrennt und dem Reaktionsgefäß wieder zugeführt,

b) aus dem Abtriebsteil wird ein Seitenabzug 8, bestehend aus Ester und Wasser, entnommen, der anschließend in dem Phasentrenngefäß 9 in die leichtere Phase 10 (in Beispiel 1 Ester) und in die schwerere Phase 11 (in Beispiel 1 Wasser) getrennt wird, wobei der Ester unterhalb der Seitenentnahme wieder in die Destillationskolonne zurückgeführt wird, und

c) als Sumpfprodukt wird der Ester 12 abgetrennt.

Die Versuchsapparatur gemäß Zeichnung bestand aus einem 2-Liter-Reaktionsgefäß 3 mit einer Verstärkungskolonne 4, deren Durchmesser 50 mm und deren Füllhöhe 600 mm betrugen. Die Füllung bestand aus 5 mm-Glasraschigringen. Die Destillationskolonne 6, deren Durchmesser ebenfalls 50 mm betrug, hatte folgende Teile, Füllhöhen und Packungsarten :

a) Verstärkungsteil, 400 mm, 5 mm Maschendrahtringe

b) oberer Abtriebsteil bis zur Seitenentnahmestelle, 1200 mm, 5 mm Glasraschigringe, und

c) unterer Abtriebsteil ab der Seitenentnahmestelle, 500 mm, 5 mm Glasraschigringe.

Beispiel 1

Bei stationärem Zustand der Anlage wurden 625 g/h Methacrylsäure und 235 g/h Frischmethanol sowie 122 g/h rückgeführtes Methanol und 17,4 g/h rückgeführtes Methylmethacrylat in das Reaktionsgefäß gefahren. Aus dem Phasentrenngefäß wurden 130 g/h Wasser, 3,6 g/h Methylmethacrylat und 2,7 g/h Methanol als untere Phase ausgetragen. Als Sumpfprodukt der 2. Destillationskolonne fielen 722 g/h Methylmethacrylat an. Das Aufteilungsverhältnis für den Seitenabzug betrug ca. 0,6. Das Rücklaufverhältnis der Verstärkungskolonne betrug ca. 0,5, das der 2. Destillationskolonne ca. 13. Die durchschnittliche Verweilzeit der Reaktionsmischung im Reaktionsgefäß lag bei 1,5 Stunden. Als Katalysator wurden 50 g Schwefelsäure eingesetzt.

Die in der Mengenbilanz fehlenden 1,7 g/h sind übliche Verluste.

**Patentansprüche**

1. Verfahren zur Herstellung von Carbonsäureestern durch Umsetzung von Carbonsäuren mit niedersiedenden Alkoholen, dadurch gekennzeichnet, daß das Reaktionsgemisch einer 1. Destillationskolonne zugeführt wird, die Reaktionskomponenten Carbonsäureester und Wasser und der nicht umgesetzte Alkohol als Kopfprodukt abgezogen und einer 2. Destillationskolonne zugeführt werden, wobei aus dieser

a) als Kopfprodukt der Alkohol oder ein azeotrop siedendes Gemisch aus Alkohol und Wasser bzw. aus Alkohol und Ester entnommen und in die Veresterungsreaktion zurückgeführt wird,

b) aus dem Abtriebsteil ein flüssiger Seitenabzug, bestehend aus Carbonsäureester und Wasser, entnommen und in einem Phasentrenngefäß in Wasser und Carbonsäureester getrennt wird und der Carbonsäureester unterhalb der Seitenentnahme der Destillationskolonne wieder zugeführt wird, und

c) als Sumpfprodukt der Carbonsäureester entnommen wird.

2. Verfahren zur Herstellung von Carbonsäureestern nach Anspruch 1, dadurch gekennzeichnet, daß man als Carbonsäuren ungesättigte Carbonsäuren mit 3 bis 7 Kohlenstoffatomen in die Veresterung einsetzt.

3. Verfahren zur Herstellung von Carbonsäureestern nach Anspruch 1, dadurch gekennzeichnet, daß man als Carbonsäuren Halogencarbonsäuren oder Mercaptocarbonsäuren mit 2 bis 5 Kohlenstoffatomen in die Veresterungsreaktion einsetzt.

4. Verfahren zur Herstellung von Carbonsäureestern nach Anspruch 1 und 3, dadurch gekennzeichnet, daß die in die Veresterung eingesetzten Halogencarbonsäuren mit ihren halogenfreien Stammverbindungen verunreinigt sind.

**Claims**

1. A process for preparing a carboxylic ester by reacting a carboxylic acid with a low-boiling alcohol, which comprises charging the reaction mixture to a 1st distillation column, withdrawing the reaction components carboxylic ester, water and unconverted alcohol as overhead product and charging this overhead product to a 2nd distillation column, from which

a) the overhead product comprises the alcohol or an azeotropic mixture of alcohol and water or alcohol and ester, this overhead product being recycled into the esterification reaction,

b) a liquid side product comprising carboxylic ester and water is withdrawn from the stripping section and separated in a phase separating vessel into water and carboxylic ester and the carboxylic ester is returned into the distillation column below the side takeoff point, and

c) the bottom product removed is the carboxylic ester.

2. A process for preparing a carboxylic ester as claimed in claim 1, wherein the carboxylic acid used in the esterification is an unsaturated carboxylic acid of from 3 to 7 carbon atoms.

3. A process for preparing a carboxylic ester as claimed in claim 1, wherein the carboxylic acid used in the esterification reaction is a halocarboxylic acid or mercaptocarboxylic acid of from 2 to 5 carbon atoms.

4. A process for preparing a carboxylic ester as claimed in claims 1 and 3, wherein the halocarboxylic acid used in the esterification is contaminated with its halogen-free parent compound.

d'halogènes.

## Revendications

1. Procédé de préparation d'esters d'acides carboxyliques par réaction d'acides carboxyliques avec des alcools à bas point d'ébullition, caractérisé en ce que l'on envoie le mélange de réaction à une première colonne à distiller d'où l'on évacue, en produit de tête, les composants du produit de réaction, ester d'acide carboxylique et eau, et l'alcool non converti, et on envoie ce produit de tête dans une deuxième colonne à distiller d'où l'on évacue :

a)   en produit de tête, l'alcool ou un mélange azéotrope d'alcool et d'eau ou d'alcool et d'ester, qu'on recycle à la réaction d'estérification,

b)   de la partie entrainement, un effluent latéral liquide consistant en ester d'acide carboxylique et eau, qu'on prépare en eau et ester d'acide carboxylique dans un récipient à séparation de phases, et on recycle l'ester d'acide carboxylique au-dessous du point de prélèvement latéral de la colonne à distiller, et

c)   en produit de pied de colonne, l'ester d'acide carboxylique.

2. Procédé de préparation d'esters d'acides carboxyliques selon la revendication 1, caractérisé en ce que l'on met en oeuvre à l'estérification, en tant qu'acides carboxyliques, des acides carboxyliques insaturés en C3-C7.

3. Procédé de préparation d'esters d'acides carboxyliques selon la revendication 1, caractérisé en ce que l'on met en oeuvre à la réaction d'estérification, en tant qu'acides carboxyliques, des acides halogénocarboxyliques ou mercaptocarboxyliques en C2-C5.

4. Procédé de préparation d'esters d'acides carboxyliques selon la revendication 1 et 3, caractérisé en ce que les acides halogénocarboxyliques mis en oeuvre à l'estérification contiennent en impuretés les acides carboxyliques correspondants exempts